# EUROPEAN PATENT APPLICATION

(11) **EP 4 356 839 A1**
(43) Date of publication of application: **24.04.2024**
(21) Application number: 22202557.9
(22) Date of filing: 19.10.2022
(51) Int. Cl.: A61B 8/00, A61B 8/08

(54) **APPARATUS AND METHOD FOR DETERMINING A MOTION PATH OF AN ULTRASOUND PROBE**

(71) Applicant: piur imaging GmbH, 1050 Vienna (AT)
(72) Inventor: Bauer, Robert, 82008 Unterhaching (DE); Linz, Lukas, 1050 Wien (AT)
(74) Representative: Zimmermann & Partner Patentanwälte mbB

(57) **Abstract**

Aspects and embodiments of the present invention relate to methods and apparatuses for determining a motion path characterizing the motion of a movable ultrasound probe across a skin surface of a body portion. An optical flow sensor fixedly mounted to the ultrasound probe generates a stream of optical flow data indicative of a two-dimensional movement of the optical flow sensor over the skin surface. A distance sensor fixedly mounted to the ultrasound probe generates distance measurement data indicative of a distance between the distance sensor and the skin surface. The motion path, determined by a pathing model, is based on the optical flow data from an optical flow sensor and the distance measurement data from the distance sensor. Further aspects relate to an ultrasound probe and a method of generating a 3D tomographic image of a body portion.

## Description

Aspects of the present application generally relate to a method and apparatus for determining a motion path of a movable ultrasound probe. The method is, in particular, carried out during acquisition of an ultrasound image of a volume portion by the ultrasound probe. Particularly, the method and apparatus may be used for the generation of ultrasound 3D tomographic images of a body portion, particularly freehand generation of ultrasound 3D tomographic images.

### Technical background:

Ultrasound imaging (ultrasound) is one of the main medical modalities for both diagnostic and interventional applications thanks to its unique properties - affordability, availability, safety and real-time capabilities. For a long time, though, it has not been possible to acquire 3D images in a simple and reliable manner, and this limitation has reduced the range of clinical applications of ultrasound. The workaround was to acquire a series of 2D images by sweeping over the region of interest and combining them into a single 3D volume afterwards.

One such implementation is, for example, described in WO 2015/191871 A1. This implementation requires a positioning system providing probe position information. External sensor-based solutions (typically using optical or electromagnetic tracking) are able to provide a good estimate of the ultrasound probe motion, and have therefore been primarily used. However, these solutions come at the expense of practicality and price.

Thus, research has been conducted for estimating the ultrasound probe motion, i.e., the relative position and orientation of the ultrasound probe from one image to the next, using additional hardware mounted to the ultrasound probe itself. Some implementations of the current state of the art include an inertial sensor mounted to the ultrasound probe to estimate the rotational movement of the transducer during the scan. However, this method is disadvantaged in that the direction of the scan must be specified before usage, as it cannot be derived from the images or the rotational data. Further, it is desired to further increase the accuracy and reliability in determining the motion, for example the position, the speed of the ultrasound probe during a scan, and/or the total distance between the start position and end position of the scan.

In view of the above, improvements to methods and apparatuses for accurately determining the motion of an ultrasound probe without external tracking are sought.

### Summary of the invention:

The present invention intends to overcome at least some of the above problems. The object is solved by the method according to claim 1, by the apparatus according to claim 12, by the ultrasound probe according to claim 15 and by the method of claim 20.

According to a first aspect of the present invention, a method of determining a motion path of a movable ultrasound probe is provided. The motion path characterizes the motion of the movable ultrasound probe across a skin surface of a body portion during acquisition of an ultrasound image of the body portion by the ultrasound probe. The method includes receiving a stream of optical flow data from an optical flow sensor fixedly mounted to the ultrasound probe, wherein the optical flow data is indicative of a two-dimensional movement of the optical flow sensor over the skin surface, receiving distance measurement data from a distance sensor fixedly mounted to the ultrasound probe, wherein the distance measurement data is indicative of a distance between the distance sensor and the skin surface, inputting the optical flow data and the distance measurement data into a pathing model, and determining, by the pathing model, the motion path based on the optical flow data and the distance measurement data.

According to a second aspect of the present invention, an apparatus for determining a motion path of a movable ultrasound probe is provided. The motion path is determined during acquisition of an ultrasound image of a body portion by the ultrasound probe. The apparatus includes an optical flow sensor for obtaining a stream of optical flow data indicative of a two-dimensional movement of the optical flow sensor over the skin surface of the body portion, a distance sensor for obtaining distance measurement data indicative of a distance between the distance sensor and the skin surface, a mounting structure for fixedly mounting the optical flow sensor and the distance sensor to the ultrasound probe, and a motion determining module configured to determine, using a pathing model, the motion path based on the optical flow data and the distance measurement data.

According to a third aspect of the present invention, an ultrasound probe configured for ultrasound imaging of a body portion is provided. The ultrasound probe includes an ultrasound transducer, an optical flow sensor configured for generating optical flow data indicative of a two-dimensional movement over a skin surface of the body portion, and a distance sensor configured for generating distance measurement data indicative of a distance between the distance sensor and the skin surface.

According to a fourth aspect of the present invention, a method of generating a 3D tomographic image of a body portion is provided. The method includes receiving a stream of ultrasound image data from an ultrasound probe while the ultrasound probe is moved across a skin surface of the body portion, receiving a stream of probe motion data of the ultrasound probe by determining a motion path according to the first aspect while the ultrasound probe is moved across the skin surface of the body portion, inputting at least a subset of the ultrasound image data representing a plurality of ultrasound image frames into a processor, inputting at least a subset of the probe motion data representing the motion of the ultrasound probe into the processor, and generating, by the processor, a 3D tomographic image based on the inputted ultrasound image data and the inputted probe motion data.

Further advantages, features, aspects and details of the invention are evident from the dependent claims, the description and the drawings.

The methods and apparatus according to aspects of the invention allows for determining the motion of an ultrasound probe using sensors, particularly an optical flow sensor and a distance sensor, which track the motion of the ultrasound probe across the surface of the skin. By relaying information about the distance and speed travelled across the skin using optical flow data, and by adjusting the optical flow data using a distance sensor, the motion of the ultrasound probe can be determined with improved accuracy. Further, apparatuses of the present invention provide reduced cost and improved practicality compared to solutions which utilise external tracking of the ultrasound probe.

### Brief description of Figures:

The invention will be better understood by reference to the following description of embodiments of the invention taken in conjunction with the accompanying drawings, wherein:
- Fig. 1a: shows schematically an ultrasound probe used in a method according to an embodiment of the invention;
- Fig. 1b: shows schematically a compounded three-dimensional ultrasound image obtained by the probe of Fig. 1a;
- Fig. 2: shows schematically details of the method for acquiring the three-dimensional image illustrated in Fig. 1a;
- Fig. 3: shows a schematic side view of an ultrasound probe according to aspects and embodiments of the present invention;
- Fig. 4a: shows schematically image data representing a plurality of ultrasound image frames, used as input in the method illustrated in Fig. 2;
- Fig. 4b: shows schematically a compounded three-dimensional ultrasound image obtained by the method illustrated in Fig. 2
- Fig. 5: shows a flow chart of a method of determining a motion path of an ultrasound probe according to aspects and embodiments of the present invention; and
- Figs. 6 and 7: show schematically neural network architectures for a machine-learning module according to respective embodiments of the invention.

### Detailed description

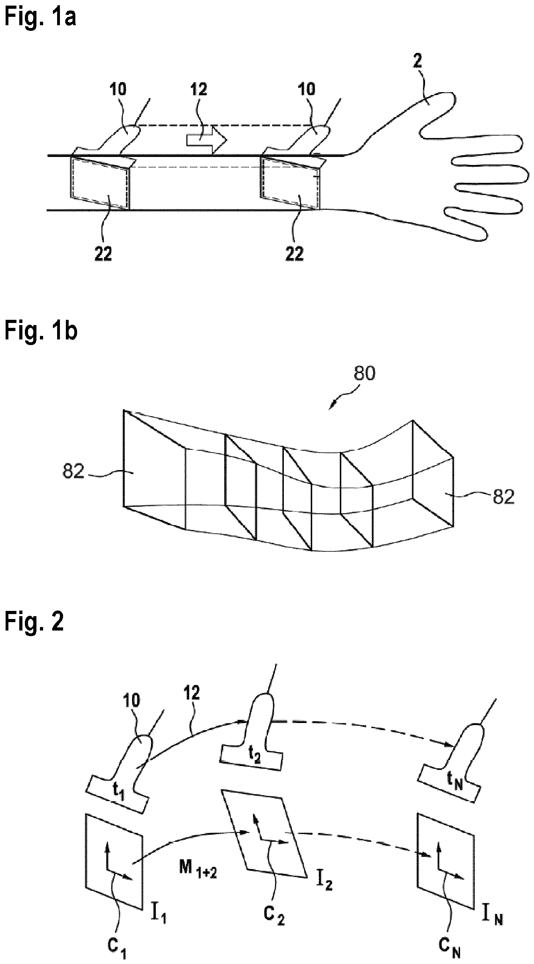

Fig. 1a shows an ultrasound probe 10 being moved along a volume portion. Here, the volume portion is a body portion 2 of a patient. The motion of the probe is indicated by a scanning direction 12 representing the motion from a starting position (probe 10 shown on the left side of Fig. 1a) to a final position of motion (probe 10 shown on the right side of Fig. 1a). During the motion, the probe 10 collects ultrasound image data representing consecutive ultrasound image frames. Each ultrasound image frame provides an ultrasound image (i.e., graphically representable information of the ultrasound reflectivity properties) in a particular imaging region or ultrasound image frame 22, i.e., in a two- or three-dimensional subspace of the body portion 2. The image frame 22 has a predetermined shape and location relative to the ultrasound probe 10, and the image frame 22 moves jointly with the ultrasound probe 10. By moving the ultrasound probe 10, the image frame 22 is moved across the body portion 2 so that the plurality of ultrasound image frames 22 provide ultrasound images of various parts of the body portion 2.

Here, an ultrasound image frame is defined as a two- or three-dimensional ultrasound image taken at a given time using the ultrasound probe. The image frame represents an entire image of a pre-defined size as acquired by the ultrasound probe. Subsequent image frames usually have the same resolution. In contrast, a dynamically selected subset of an ultrasound image frame, selected in dependence of the image content and possibly with variable size, is not an image frame. Typically, a time stamp is associated with the ultrasound image frame. The probe 10 collects the ultrasound image data as a data stream representing consecutive ultrasound image frames.

Fig. 1b schematically shows an output of aspects of the present invention, a compounded three-dimensional ultrasound image, otherwise referred to as a 3D tomographic image. The compounded three-dimensional ultrasound image is a three-dimensional image indicating the ultrasound reflectivity properties in the scanned body portion, obtained from the acquired ultrasound image frames and the determined movement (position and optionally orientation) of the ultrasound probe 10 for each of the acquired ultrasound image frames 22. The compounded three-dimensional ultrasound image can, for example, be visualized as the set of the image frames positioned in space, i.e. a determined spatial arrangement of image frames 82 arranged along a probe trajectory 80. Alternatively, the compounded three-dimensional ultrasound image can be visualized as a full 3D image, if further processed with a compounding algorithm such as the 3D reconstruction described further below.

Fig. 2 depicts in more detail the challenging technical problem that aspects and embodiments of the present invention aim to solve. During the acquisition, the ultrasound probe (10) is moved and the image content of the image frames 22 is therefore changing. An object of the present invention is to determine the motion path of the probe between two instants t1 and t2. In examples disclosed herein, the motion path can be represented as a matrix M₁₂ that models the relative transformation between the coordinate system of one frame C₁ and the coordinate system of the other frame C₂. This process can then be repeated for the whole series of images. Typically, the motion has six degrees of freedom (three translations and three rotations), and the matrix M₁₂ can be parametrized by 6 parameters. Based at least on matrix M₁₂ and the plurality of ultrasound image frames, the generation of 3D tomographic images is possible. For this purpose, matrix M₁₂ may be derived from a motion path which characterizes a motion of the ultrasound probe, i.e. the probe trajectory, as the ultrasound probe 10 is moved across the body portion 2. It follows that accurate derivation of the motion path is desirable. The objective of the aspects and embodiments of the present invention is to determine the motion of an ultrasound probe with improved accuracy, improved practicality compared to an externallytracked system, and with reduced cost.

Various objectives of the present invention were identified. It is desirable for the method and apparatus to have a low cost of implementation and low power consumption. Further, it is desirable for the apparatus to have a compact size so that the apparatus may be part of or mounted to the ultrasound probe without affecting usability. Further, the system should be able to be mounted to a hand-held ultrasound probe, which restricts the operating distance of any sensors to near-field operation only.

In the following disclosure, aspects and embodiments of the invention are defined in detail. Each aspect so defined may be combined with any other embodiment or with any other aspect(s) unless clearly indicated to the contrary. Reference signs referring to the Figures are for illustration only, but are not intended to limit the respective aspect(s) to the embodiments shown in the Figures.

### Main Embodiment

The present invention aims to solve the problems and disadvantages identified in the state of the art by implementing a combination of sensors to be attached to an ultrasound probe for determining a motion of the probe during acquisition of an ultrasound image. Particularly, the sensors attached to the ultrasound probe allow for a so-called "inside-out" determination of the motion, i.e. without the need for tracking using external sensors. The present invention achieves this objective by the acquisition of optical flow data from an optical flow sensor directed to observe the skin surface of the body portion, so that the optical flow data is indicative of a two-dimensional movement of the optical flow sensor - and thereby of the ultrasound probe to which it is fixed - over the skin surface (2a). Through subsequent processing of the optical flow data, a motion path may be determined which corresponds at least to a two-dimensional path along the surface of the body portion.

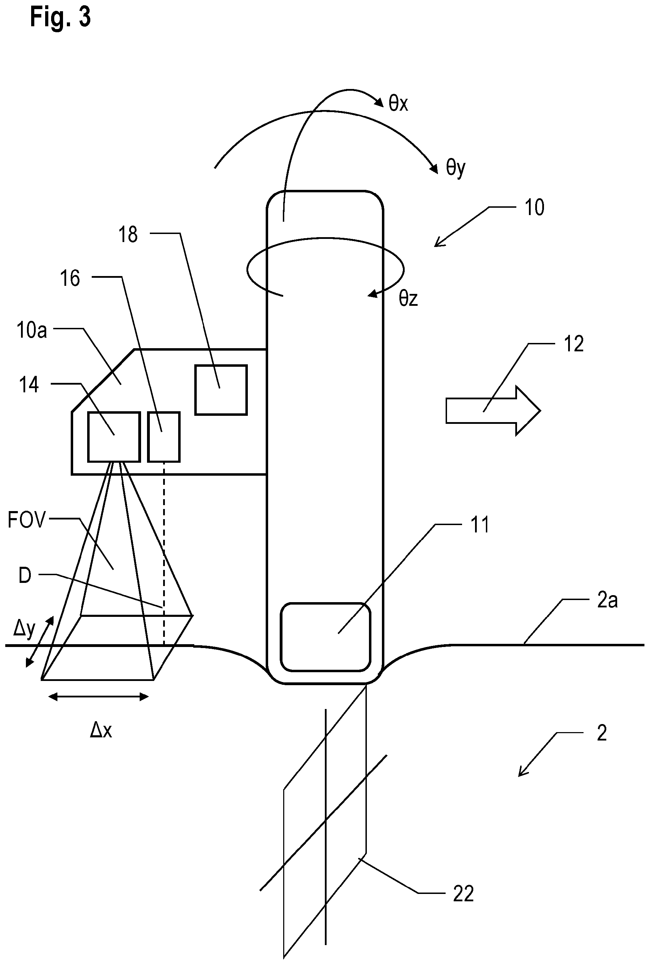

However, referring to Fig. 3, further challenges arise with the use of an optical flow sensor for detecting optical flow on a skin surface. The exemplary ultrasound probe 10 shown in the figure includes optical flow sensor 14, which is arranged at a distance from the skin surface. Challenges arise due to the skin surface being deformable. As the ultrasound probe is scanned over the skin surface in a scanning direction 12, the transducer end of the ultrasound probe 10 may be pressed into the surface of the skin, changing the distance between the optical flow sensor and the skin surface. Further, challenges associated with the use of a hand-held ultrasound probe also arise, as the operator is unlikely to scan the ultrasound probe across the skin surface at an exact perpendicular orientation to the skin surface. A roll, yaw or pitch of the ultrasound probe may also change the distance between the optical flow sensor and the skin surface.

The optical flow data indicates the two-dimensional movement of the ultrasound probe only in terms of pixels. For determining the motion path of the ultrasound probe in physical units such as mm moved along the surface, the optical flow data indicating the two-dimensional movement is not sufficient, but also knowledge of the distance between the optical flow sensor and the skin surface is required. Stated differently, any variation of the distance between the optical flow sensor and the skin surface may affect the motion path (in physical units) that corresponds to a given optical flow data generated by the optical flow sensor.

In view thereof, the present invention further includes a distance sensor 16 configured for measuring distance measurement data. The distance measurement data is indicative of a distance D between the distance sensor 16 and the skin surface 2a, which contains information also about the distance between the optical flow sensor 14 and the skin surface 2a (since both sensors 14 and 16 are fixed to each other and to the ultrasound probe).

Information about this distance is obtained from the distance sensor 16, which is also fixedly mounted to the ultrasound probe 10. The distance sensor 16 outputs distance measurement data indicative of a distance D between the distance sensor 16 and the skin surface 2a, and thereby also contains information regarding the distance between the optical flow sensor and the skin surface.

The motion path of the ultrasound probe (in physical units) is thus obtained by inputting the optical flow data from the optical flow sensor 14 and the distance measurement data from the distance sensor 16 into a pathing model, which then outputs the motion path (in physical units). In an example, the pathing model contains the operations of
(i) Determining the distance between the optical flow sensor 14 and the skin surface 2a based on the distance D between the distance sensor 16 and the skin surface 2a (this operation may be the identity operation in case both sensors 14 and 16 are mounted at the same height from the skin surface 2a, and may include adding a height offset between the two sensors in case both sensors 14 and 16 are not mounted at the same height from the skin surface 2a);
(ii) Multiplying the optical flow data (indicating a motion in pixels) by the distance determined in step (i) and optionally by a scaling factor (e.g., empirically determined in an initial calibration step).

The pathing model thus allows for the raw two-dimensional motion data detected by the optical flow sensor to be appropriately scaled, transformed, modelled or adapted based on the distance, so that an accurate motion path may be determined. By supporting the optical flow data with distance measurement data, any variation in the distance caused by e.g. rotation of the ultrasound probe 10 or deformation of the skin surface 2a may be accounted for to accurately and reliably determine the motion path of the ultrasound probe 10.

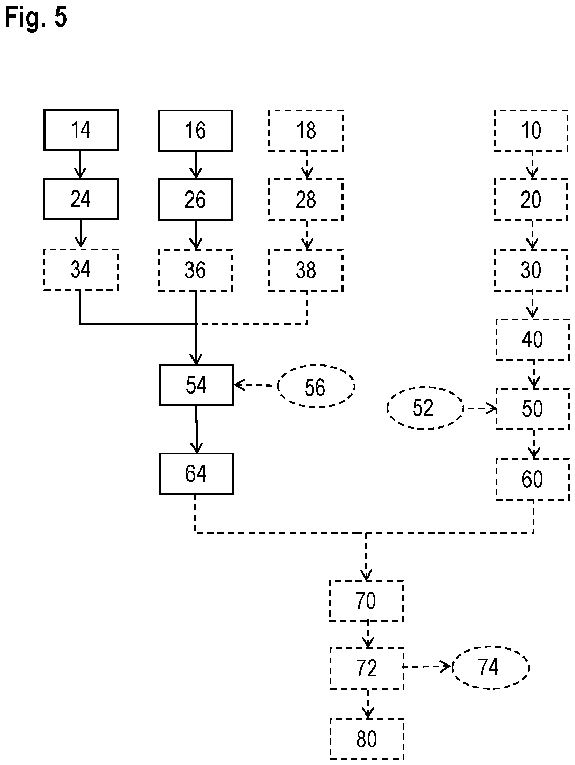

Reference will now be made to Figs. 3 and 5, which show an exemplary ultrasound probe 10 and a flow chart of a method of determining a motion path 64 of the ultrasound probe 10. According to an aspect of the present invention, a method of determining a motion path 64 of a movable ultrasound probe 10 is provided. The motion path 64 characterizes the motion of the ultrasound probe 10 across a skin surface 2a of a body portion 2 during acquisition of an ultrasound image of the body portion 2 by the ultrasound probe 10. The method according to this aspect includes receiving a stream of optical flow data 24 from an optical flow sensor 14 fixedly mounted to the ultrasound probe 10, wherein the optical flow data 24 is indicative of a two-dimensional movement of the optical flow sensor 14 over the skin surface 2a. Next, the method includes receiving a distance measurement data 26 from a distance sensor 16 fixedly mounted to the ultrasound probe 10, wherein the distance measurement data 16 is indicative of a distance D between the distance sensor 16 and the skin surface 2a. The optical flow data 24 and the distance measurement data 26 are input into a pathing model 54 and, by using a pathing model 54, the motion path 64 is determined based on the optical flow data 24 and the distance measurement data 26.

First, aspects of the optical flow sensor 14 are described. The type of optical flow sensor implemented in the invention has a significant effect on achieving the objectives mentioned above. The inventors investigated various types of optical flow sensors to find a suitable sensor for the present invention, and four types of sensor were evaluated, including a light detection and ranging (LiDAR) sensor, a radar sensor, a camera and an optical tracking sensor.

Light detection and ranging (LiDAR) sensors use pulsed lasers that reflect from the surfaces of objects, allowing for a distance value to be measured. With repeated reflection and recording of laser pulses, a map of the surface can be generated, and movement of the surface may be measured as optical flow data by comparing the maps in a time series. Although a LiDAR sensor has the advantage of low computational power and does not require external illumination of the skin surface, LiDAR sensors are typically expensive, large in size and unsuitable for near-field applications.

Radar sensors were also considered for the present invention. Similarly with LiDAR sensors, a radar sensor may be used for repeated reflection and recording of radio waves for generating a map of the surface, and movement may be detected by comparing maps in time series. However, a radar sensor would not be suitable for near-field operation, has a low resolution, and may have an effect on the ultrasound transducer in the ultrasound probe.

One suitable option is a camera, optionally provided with an illuminating light source. The use of an image sensor of a camera allows for movement estimation based on optical flow by determining the relative movement between two consecutive images in a sequence. A camera with optional illuminating light source allows for flexible implementation, is readily available for low cost, and has a compact size for mounting to an ultrasound probe. Despite minor disadvantages being evident in terms of higher computational requirements and higher power draw, the implementation of a camera as the optical flow sensor is a suitable option for the present invention.

A further suitable option is an optical tracking sensor. Although similar to a camera, an optical tracking sensor includes integrated logic which converts the recorded data into a stream of two-dimensional motion data across the surface in the x-direction and the y-direction. The integrated logic converts the data at high speed and with high accuracy, with low computational requirements and low power draw. Optical tracking sensors are also sufficiently compact to be mounted to an ultrasound probe.

Thus, according to an embodiment, which may be combined with embodiments and aspects described herein, the optical flow sensor is one of the group comprising an optical tracking sensor and a camera.

An optical flow sensor, particularly an optical tracking sensor, may be configured to generate data indicative of position differential data Δx, Δy relative to the skin surface, which indicates a relative distance moved within the previous sample interval. Further, an optical tracking sensor may be configured to output data indicative of position summation data Σx, Σy relative to the skin surface, e.g. a summation of the differential data Δx, Δy, which indicates a total distance moved in a certain time frame. For example, the position summation data Σx, Σy may indicate the total distance moved since the optical flow sensor was last reset or zeroed.

Different optical flow sensors are available for a variety of applications. One common type of optical flow sensor in the current state of the art is an optical tracking sensor for a computer mouse, such as a laser optical mouse sensor. However, a typical computer mouse sensor is configured for operating in very near-field applications at a small working height. The term "working height" in the context of the present disclosure relates to an operating range of the distance from the surface being measured and the optical tracking sensor. An optical tracking sensor in a computer mouse has a very small working height to the surface, e.g. at most 1 mm. At larger working distances, i.e. in excess of a so-called "lift-off distance", the optical tracking sensor of the computer mouse no longer detects movement. Since a small deformation of the skin surface, or a small rotation of the ultrasound probe, would move the optical flow sensor outside of such a narrow working height range, an optical tracking sensor for computer mice would not be suitable for the present invention due to the limited operation range from the surface of the skin.

However, other types of optical flow sensors with a larger working height are available for use in autonomous vehicles and robotics. Ground-based, small autonomous vehicles such as small utility robots, robotic vacuum cleaners and robotic lawn mowers may use optical tracking sensors for detecting speed and direction across a surface upon which the vehicle is driving. Such optical tracking sensors typically operate with a near-field working distance of up to 120 mm, and are highly suitable for the present invention. An example of a suitable optical tracking sensor is the model PAA5100JE-Q optical tracking chip, available from manufacturer PixArt Imaging Inc.

The model PAA5100JE-Q sensor has a working height in the range of 15 mm to 35 mm. However, the present invention is not limited thereto, and the optical flow sensor may have a working height in the range of more than 0 mm. The upper limit of the working height range may be limited by the length of the ultrasound probe. More particularly, the working height of the optical flow sensor may be in the range of up to 120 mm. Preferably, the working height of the optical flow sensor may be at least 1 mm, or more preferably at least 5 mm. Further, the working height of the optical flow sensor may be at most 60 mm, more preferably at most 35 mm. For example, the optical flow sensor may have a working height in the range between at least 15 mm and at most 35 mm.

The optical flow sensor 14 is fixedly mounted to the ultrasound probe 10 so that the movement of the ultrasound probe 10 results in a corresponding movement of the optical flow sensor 14. In the context of the present disclosure, the term "fixedly mounted" relates to the fixed relative arrangement of a component with respect to another. For a component to be "fixedly mounted" to another, there is no relative spatial movement or rotation between the respective components and the rotation and/or movement of one component implies the same rotation and/or movement is experienced by the other component mounted thereto. However, the term "fixedly mounted" does no limit the means of fixing the components together. For example, the components may be mounted in the same housing, may be in separate housings designed to be connected together, may be mounted together with the use of adhesive, or may be configured to be mounted and demounted in a temporary manner. Also, the fixing can be temporary, e.g., by using a clip-on mechanism designed to create a well-defined, rigid spatial relation between the components.

The optical flow sensor 14 is arranged at an offset distance from the longitudinal axis (i.e. the z-axis) of the ultrasound probe 10 so that the optical flow sensor 14 may have a field of view (FOV) which enables the measurement of optical flow data across the skin surface 2a. The offset distance from the longitudinal axis of the ultrasound probe 10 further allows for the optical flow sensor to detect a yaw rotation θz of the ultrasound probe 10.

The field of view (FOV) (i.e. the effective viewing angle) of the optical flow sensor may be selected so as to be as large as practical, but without being occluded by an object such as the hand of the operator or the body of the ultrasonic probe 10. In other words, the optical flow sensor may have a field of view (FOV), and be arranged such that only a portion of the skin surface 2a is visible to the sensor within the field of view (FOV). An effective viewing angle of the optical flow sensor, in the context of the present disclosure, is defined as the total angle of the cone of vision forming the field of view (FOV), and corresponds to double the angle from the cone of vision to the direction normal to the optical view sensor. The effective viewing angle may be in the range of 10° to 60°. Preferably, the effective viewing angle may be in the range of 30° to 50°. The example model PAA5100JE-Q sensor discussed above has an effective viewing angle of 42°.

Further performance specifications of the optical flow sensor may include frame rate and resolution. For example, a high frame rate optical flow sensor may have improved performance in tracking the surface of the skin. The example model PAA5 100JE-Q sensor discussed above has a frame rate of 242 frames per second. Preferably, the optical flow sensor may have a frame rate of at least 60 frames per second, more preferably at least 120 frames per second, and/or up to 480 frames per second.

Further, the performance of the optical flow sensor may be improved by external illumination. For example, the optical flow sensor may be enhanced with the addition of one or more lighting elements, particularly one or more LEDs, for illuminating the surface of the skin for improved optical tracking. The illumination can be with visible light but also with infrared light, for example.

Next, aspects of the distance sensor 16 are described. Distance sensor 16 is fixedly mounted to the ultrasound probe 10 and is directed towards the skin surface 2a so as to measure a distance D between the distance sensor 16 and the skin surface 2a. The distance D is measured as the distance between the distance sensor 16 and the skin surface 2a, however due to the optical flow sensor 14 also being fixedly mounted to the ultrasound probe 10, the distance D measured by the distance sensor 16 also is also indicative of the distance between the optical flow sensor 14 and the skin surface 2a, e.g. with a known height offset.

The distance sensor 16 generates distance measurement data 26 which can be used, in combination with the optical flow data 24, to determine a motion path 64 of the ultrasound probe 10 while accounting for changes in the distance between the respective sensors and the skin surface 2a. For example, in the case where, during scanning of the ultrasound probe 10 in the scanning direction 12, the ultrasound probe 10 is pressed into the body portion 2 to cause a deformation of the skin surface 2a, the distance sensor 16 is configured to measure the distance D and generate the required distance measurement data 26 to compensate for the deformation. Similarly, in the case where, during scanning of the ultrasound probe 10 in the scanning direction 12, the ultrasound probe 10 is rotated by the operator in at least one of a pitch motion θy and a yaw motion θz, the generation of the distance measurement data 26 allows for the rotation to be compensated. Thus, the determination of the motion path 64 may be achieved with improved accuracy and reliability.

The distance measurement data 26 generated by distance sensor 16 may be further used for determining whether the optical flow sensor 14 is operating within the specified working height range of the optical flow sensor 14. For example, if the distance sensor 16 senses a distance D within the specified working height range, the optical flow data 24 may be considered to be valid for determining the motion path 64. Conversely, if the distance sensor 16 senses a distance D which is outside of the specified working height range, the optical flow data 24 may be partially vetoed, flagged for partial error correction, or partially substituted with motion data from another source.

Distance sensor 16 is arranged to sense the distance to the skin surface 2a. So that the any deformation of the skin surface 2a may be compensated, it is preferable that the distance sensor 16 measures the distance to the skin surface 2a at a portion of the skin surface 2a which is offset from the ultrasound probe 10, particularly the operating end of the ultrasound probe 10. More particularly, it may be preferable to arrange the distance sensor 16 so that the distance to the skin surface 2a is measured to a portion of the skin surface 2a which is contained within the field of view (FOV) of the optical flow sensor 14. Such a preferable arrangement, while not strictly essential, would allow for a closer correlation of the values of the distance D measured between the distance sensor 16 and the skin surface 2a and the actual distance between the optical flow sensor 14 and the skin surface 2a.

For aspects and embodiments of the present invention, the distance sensor 16 may be any distance sensor known in the state of the art which is suitable for measuring a distance to a skin surface. The distance sensor 16 should preferably be capable of measuring distance to a skin surface which may be coated with ultrasound transmission gel. It is preferable that the distance sensor 16 is a non-contact distance sensor, more particularly an optical time-of-flight sensor. More preferably, the distance sensor is a laser distance sensor which determines a distance based on the time-of-flight of a reflected laser pulse.

Further, in order to detect whether the optical flow sensor 14 is working outside of the specified working height range, the distance sensor 16 should have a working height range at least broader than the working height range of the optical flow sensor 14. The distance sensor 16 may have, for example, a working height range of up to 150 mm, more particularly up to 120 mm.

As an example, a model VL6180X time-of-flight LiDAR distance sensor from the manufacturer STMicroelectronics NV may be used, which has a working height range of 5 mm to 100 mm. However, the present invention is not limited thereto.

Next, aspects of the pathing model 54 are described. The pathing model 54 is configured to receive optical flow data 24 generated by the optical flow sensor 14 and distance measurement data 26 generated by the distance sensor 16 as inputs. Using the pathing model 54, the motion path 64 of the ultrasound probe 10 may be determined based on the data input into the pathing model 54.

The pathing model 54 may include any model configured to transform, scale, substitute, verify, compare or correct the optical flow data 24 to determine a motion path 64 based thereon. Particularly since the optical flow data 24 may be affected by the distance of the optical flow sensor 14 from the skin surface 2a, the optical flow data 24 may be manipulated by the pathing model 54 to determine an accurate motion path 64.

At the most general level, the optical flow data 24 generated by the optical flow sensor 14 may change depending on the working height of the optical flow sensor 14. For example, for the example model PAA5100J-E optical tracking sensor, the counts per inch value output from the sensor will vary depending on the distance of the sensor from the skin surface 2a. The optical flow data 24, e.g. the counts per inch values in at least the x-direction and the y-direction, may be manipulated by the pathing model 54 to compensate for this variability. Generally, the pathing model 54 converts the raw optical flow data 24 into a motion path 64 which is indicative of the actual motion of the ultrasound probe 10.

In a form of least complexity, the pathing model 54 may be a linear scaling model in which the position differential data Δx, Δy is linearly scaled based on the distance measurement data 26 generated by the distance sensor 16. The scaling coefficients may be based on empirical data obtained from experimentation. A simple linear scaling model may be sufficient for ultrasound scans which are sufficiently linear across the skin surface 2a. Similarly, the pathing model 54 may include a non-linear scaling model in which the position differential data Δx, Δy is nonlinearly scaled based on the distance measurement data 26 generated by the distance sensor 16.

The pathing model 54 may alternatively be based on an empirical model generated from data obtained in experimental testing. For example, the pathing model 54 may include a lookup table of scaling coefficients for scaling or transforming the optical flow data 24. The lookup table may be based on the distance measurement data 26, or other variables such as a scanning velocity or scanning acceleration.

As a further alternative, the pathing model 54 may include a machine learning model which is trained to determine a pathing model 64 based on at least the optical flow data 24 and the distance measurement data 26. The machine learning model may be trained by and/or based on training data 56 obtained from experimental testing.

The pathing model 54 may be configured to transform the optical flow data 24 based on, for example, one or more determined rotations. In the case of a roll rotation θx of the ultrasound probe 10, i.e. when the ultrasound probe 10 may be tilted sideways with respect to the scanning direction, the portion of the skin surface within the field of view (FOV) of the optical flow sensor 14, i.e. a measurement window, may be skewed. The pathing model 54 may perform a mathematical transformation to correct for the skewed measurement window, e.g. an affine transformation.

The method of determining the motion path 64 may further include substituting optical flow data 24 with data from other sources. The substituting may be performed in any one of a pre-processing performed in an optical flow pre-processing module 34, the pathing model 24, or by a post-processing performed in a post-processing module 70. For example, if the distance measurement data 26 indicates that the optical flow sensor 14 may be operating outside of a specified working height range, the optical flow data 24 may be partially replaced with movement data from another source.

Similarly, the method may further include verifying or comparing the optical flow data 24 to data obtained from other sources. Based on the verifying or comparison, the optical flow data 24 may be further corrected to account for errors. Generally, the merging of the motion path 64 and the image motion path 60 may include verifying 72 a consistency condition 74 between the motion path 64 and the image motion path 60.

According to an embodiment, which may be combined with aspects and embodiments described herein, the pathing model 54 includes at least one of the group consisting of a linear scaling model, a non-linear scaling model, an empirical model based on empirical data, and a machine learning model.

The motion path 64 may be defined as a set of data which describes the path undertaken by the ultrasound probe 10 during acquisition of ultrasound image data as the ultrasound probe 10 is scanned across the skin surface 2a of a body portion 2. The motion path 64 may include a number of motion variables which may describe the spatial position, spatial orientation or spatial velocity of the ultrasound probe 10. Particularly, the motion path 64 may be defined as a spatial motion with respect to the skin surface 2a. More particularly, during acquisition of a plurality of ultrasound image frames 22, the motion path 64 is indicative of a position and orientation of each ultrasound image frame 22 respective previous or subsequent ultrasound image frames 22. The motion path 64 is given in physical units (in particular in units that do not scale with the distance of the optical sensor 14 from the skin surface 2a)

In addition, the motion path 64 may also comprise metadata, e.g. indicating the type of optical flow sensor, markers indicating positions and/or times at which ultrasound image frames are acquired, or time synchronization data. The metadata may also be partially or fully provided as a time series.

The motion path 64 may be defined with varying amounts of variables, e.g. depending on which type of body portion 2 is being scanned. For example, when scanning across a relatively linear and flat body portion, e.g. an arm or leg, the motion path 64 may be defined with reduced complexity using a scanning direction and a scanning distance of the ultrasound probe 10 with respect to the skin surface 2a. Since the skin surface 2a of such a linear, flat body portion 2 does not include significant curvature, the motion path 64 may be sufficiently accurate when defined in terms of the two-dimensional (or even one-dimensional) motion of the ultrasound probe 10 relative to the skin surface 2a.

A scanning direction may correspond to a direction determined from the optical flow data, particularly the position differential data Δx, Δy output from the optical flow sensor to determine a direction vector in which the ultrasound probe 10 is being moved relative to the skin surface 2a. A scanning distance may correspond to a summation of the position differential data Δx, Δy, or may be obtained from summation data Σx, Σy output from the optical flow sensor.

Further optional variables may be determined to improve accuracy of the motion path 64 in other situations. The further optional variables described in the following may be determined by any one of a pre-processing performed in an optical flow pre-processing module 34, the pathing model 24, or by a post-processing performed in a post-processing module 70. For example, a yaw motion of the ultrasound probe may be determined with respect to the skin surface 2a. For example, when the scanning direction 12 is substantially in the y-direction, the yaw motion may be determined based on the optical flow data in the x-direction, indicating a yaw of the ultrasound probe 10. If the ultrasound probe 10 is rotated at an angle in the yaw direction when a respective ultrasound image frame 22 is acquired, the resulting image frame will also be rotated. Determining a yaw motion and including the yaw motion in the motion path 64 allows for improved accuracy in locating the position and orientation of the resulting ultrasound image frames 22. Similarly, the motion path 64 may also include other rotational motion of the ultrasound probe 10, such as a roll motion or a pitch motion. Generally, the motion path 64 may include data indicative of position and rotation in up to six degrees of freedom (DOF). For example, the motion path 64 may include at least one of the group comprising a roll motion θx, a pitch motion θy and a yaw motion θz of the ultrasound probe 10 with respect to the body portion 2.

Optionally, the motion path 64 may include data indicative of a scanning depth with respect to the body portion 2. As discussed above, the skin surface 2a of the body portion 2 may deform with varying pressure exerted on the ultrasound probe 10. Any one of the pre-processing performed in the optical flow pre-processing module 34, the pathing model 24, or the post-processing performed in the post-processing module 70 may be configured to account for varying scanning depth, i.e. varying amounts of deformation of the skin surface 2a, based on the distance measurement data 26 generated by the distance sensor 16.

Optionally, the motion path 64 may include data indicative of a forward-backward directedness. A forward-backward directedness corresponds to an indication of whether the ultrasound probe is being scanned in a forward direction or a backward direction. For example, the forward-backward directedness may indicate whether the ultrasound probe is being scanned away from the starting point (forward) or towards the starting point (backward).

In view of the above, according to embodiments which may be combined with aspects and embodiments described herein, the motion path 64 includes at least one of the group comprising a scanning direction of the ultrasound probe 10 with respect to the skin surface 2a, a scanning distance of the ultrasound probe 10 with respect to the skin surface 2a, a yaw motion of the ultrasound probe 10 with respect to the skin surface 2a, a scanning depth with respect to the body portion 2, and a forward-backward directedness.

Optionally, the optical flow data 24 generated by the optical flow sensor 14 may be pre-processed by the optical flow pre-processing module 34. Further, the distance measurement data 26 generated by the distance sensor 16 may similarly be pre-processed by a distance measurement data pre-processing module 36. The optical flow data 24 and/or distance measurement data 26 may be pre-processed in the respective pre-processing modules 34, 36 with a variety of algorithms, such as noise filtering, smoothing, interpolation or other high-level analysis.

The determining of the motion path 64, i.e. including the pathing model 54, may be implemented on a motion determining module. The motion determining module may include a controller, a microprocessor, a programmable logic controller (PLC), or a digital signal processor (DSP). Particularly, the motion determining module may include a processing element, at least one input and at least one output, such that a data processing operation is performed on the at least one input and output to the at least one output. The motion determining module may further include at least one storage means, which may include random access memory (RAM), read-only memory (ROM) and external data storage means such as hard disks, flash storage or network-attached storage. The motion determining module may further include a wired or wireless data connection for interfacing with a data network, particularly for transmitting the resulting motion path 64 to another module, system or apparatus for performing processing based on the motion path 64.

As a preferable arrangement, the motion determining module is implemented as a microcontroller fixedly mounted to the ultrasound probe 10. For example, the microcontroller may be integrated into a sensor unit having a mounting structure 10a for attaching to the ultrasound probe 10. The microcontroller would further include a wired or wireless transmitting means for transmitting the motion path 64 to an external processing apparatus, e.g. for generating the 3D tomographic image of the body portion 2. Example processing devices may include an Arduino microcontroller, an ESP32 microcontroller or a Raspberry Pi single-board computer (SBC). The device is programmed with the pathing model 54 for determining the motion path 64, and includes Bluetooth or WLAN for transmission of the motion path data.

Alternatively, the motion determining module may be an external processing module which is located externally to the ultrasound probe 10. For example, the module, system or apparatus for generating the 3D tomographic image may include the motion determining module, and may be configured to receive optical flow data 24 and distance measurement data 26 from the respective sensors with a wired or wireless transmission means.

An example system is described in the following, however the present disclosure is not limited thereto. The apparatus for determining a motion path includes the sensors described herein, including an optical flow sensor, a distance sensor, and optionally an inertial measurement unit. The sensors report their respective data to a microcontroller, for example an ESP32 microcontroller. The respective data is then sent wirelessly (e.g. via Bluetooth or WLAN) to a computer, or optionally, first to a Piur Imaging GmbH Infinity Box and then to a computer. In the example system, the respective data from the distance sensor and the optional inertial measurement unit do not require processing, as the respective sensors report the respective data in a suitable raw form. The data reported form the optical flow sensor is processed for conversion into millimetres for the differential data Δx, Δy and summation data Σx, Σy. Said processing for the optical flow sensor data is preferably carried out on the computer, or may optionally be carried out on the microcontroller. The resulting pre-processed data may be computed by a pathing model on the computer to determine a motion path, and then supplied to a convolutional neural network (CNN, described below) for further processing.

### First Extension Embodiment

According to an embodiment of the present invention, the method of determining a motion path 64 may be further enhanced by the acquisition of inertial data. An inertial measurement unit 18 may optionally be mounted to the ultrasound probe 10 to further augment the sensor data acquired for determining the motion of the ultrasound probe 10. The inertial measurement unit 18 may be configured to generate inertial data 28 indicative of a rotation of the ultrasound probe.

According to an embodiment, which may be combined with other embodiments described herein, the method of determining a motion path 64 further includes receiving inertial data 28 from an inertial measurement unit 18 fixedly mounted to the ultrasound probe 10, wherein the inertial data 28 is indicative of a rotation θx, θy, θz of the ultrasound probe 10. The determination of the motion path 64 by the pathing model 54 is further based on the inertial data 28.

Referring once again to Figure 5, the overall workflow of the first extension embodiment is shown, with the optional steps indicated with dashed lines now being included in the overall method. The inputs of the method for determining the motion path 64 (shown on the left side of the flow-chart) is the optical flow data 24 generated by the optical flow sensor 14, the distance measurement data 26 generated by the distance sensor 16, and the inertial data 28 generated by the inertial measurement unit 16. The inertial data 26 may be pre-processed with a variety of algorithms in an image data pre-processing module 38, such as noise filtering, smoothing, interpolation or other high-level analysis. The inertial data 28, along with the optical flow data 24 and the distance measurement data 26, can then be input into the pathing model 54 to produce a motion path 64 characterizing the motion of the ultrasound probe 10 as the probe is moved across the skin surface 2a during acquisition of the ultrasound image. Optionally, the merged motion path data is then post-processed in a post-processing module 70 to produce the final trajectory of the probe 80.

According to embodiments, the inertial data 28 may include a roll motion θx, defined as a rotation about the x-axis which is arranged along the skin surface 2a approximately in line with the direction of motion of the ultrasound probe 10. In the case of a roll motion θx, the optical flow sensor 14 may detect negligible optical flow in either the x-direction Δx or the y-direction Δy, and the additional input afforded by the inertial measurement unit 18 may improve tracking accuracy for probe roll motion.

Inertial data 28 may further include a pitch motion θy, defined as a rotation about the y-axis which is arranged along the skin surface 2a approximately perpendicular to the direction of motion of the ultrasound probe 10. In the case of a pitch motion θy, the distance D between the distance sensor 16 and the skin surface 2a will vary. This pitch motion may be detected by the distance sensor 16, and as such, the additional input for tracking probe pitch motion is not essential. However, in the case where the ultrasonic probe 10 may be pitched excessively such that the optical flow sensor 14 would be operating outside of the specified working height range, the additional input offered by the inertial measurement unit 18 may be used for correction of the optical flow data, verification of the optical flow data, or substitution of the optical flow data to further improve tracking accuracy for probe pitch motion.

Inertial data 28 may further include a yaw motion θz, defined as a rotation about the z-axis which is arranged approximately perpendicular to the skin surface 2a. In the case of a yaw motion θz, the offset of the optical flow sensor 14 from ultrasound probe 10 causes the optical flow sensor 14 to detect an optical flow across the skin surface 2a in the y-direction Δy. This yaw motion may therefore be indicated by the optical flow data, and as such, the additional input for tracking probe yaw motion is not essential. However, the additional input offered by the inertial measurement unit 18 may be used for further improving tracking accuracy for probe yaw motion, particularly in a case where a yaw motion is combined with a pitch motion causing the optical flow sensor 14 to be operating outside of the specified working height range.

The pathing model 54 may be configured to determine the motion path 64 based on the inertial data 28 in many ways. The pathing model 54 may be configured to perform verification, scaling, transformation, correction, or substitution of the optical flow data 24. For example, if the distance measurement data 26 indicates to the pathing model 54 that the optical flow sensor 24 is operating outside of the specified working height range, the pathing model 54 may substitute a portion of optical flow data 24 with data based on the inertial data 28 to determine a portion of the motion path 54. As a further example, if the optical flow data 24 indicates to the pathing model 54 that a pitch motion θy or a yaw motion θz of the ultrasound probe 10 is detected, the pathing model 54 may verify the pitch motion θy or yaw motion θz based on the inertial data 28. As yet a further example, if the inertial data 28 indicates a rotation θx, θy or θz of the ultrasound probe 10 which may cause the field of view FOV of the optical flow sensor 14 to be skewed, distorted or otherwise transformed, the pathing model 54 may scale or transform the optical flow data 24 based on the inertial data 28 and a mathematical or empirical model.

The method according to the first extension embodiment exhibit advantages over the current state of the art which track the ultrasound probe using only inertial data. Accordingly, the merging of the optical flow data 24, distance measurement data 26 and inertial data 28 allows for improved accuracy in tracking the motion of the ultrasound probe, while also overcoming the disadvantages of a purely inertial-based system. Particularly, the present embodiment does not require a scanning direction to be specified before usage, allows for improved accuracy in determining the speed of the ultrasound probe during a scan, and allows for improved accuracy in determining the total distance between the start position and end position of the scan.

The inertial data 28 generated by the inertial measurement unit 18 is not limited to inertial data indicative of a rotation. Inertial measurement unit 18 may further include at least one accelerometer for providing inertial data indicative of an acceleration in at least one direction. For example, the inertial data 28 may include at least one of an acceleration in the x-direction, an acceleration in the y-direction and an acceleration in the z-direction. Acceleration data may be integrated to determine velocity and distance of the ultrasound probe for verification, scaling, transformation, correction or substitution of the respective velocity or distance data measured by the optical flow sensor 14.

### Second Extension Embodiment

According an embodiment of the present invention, the method of determining a motion path 64 may be further enhanced by merging the motion path 64 with additional estimations determined from the ultrasound image frames 22 acquired by the ultrasound probe 10. In this embodiment, a second motion path derived from the ultrasound image frames 22, hereafter referred to as an image motion path 60, can be merged with the motion path 64 determined from the sensor arrangement either by comparison, by verification, or my superposition through a further pathing model. As a result, further improvements to the accuracy of the final trajectory data 80 are possible.

Reference is made to European patent application number EP 3 522 789 A1, which is hereby incorporated by reference, describing the method of determining motion of an ultrasound probe based on the relative three-dimensional motion between the ultrasound image frames acquired by the ultrasound probe.

According to an embodiment, which may be combined with other embodiments described herein, the method of determining a motion path 64 further includes receiving a stream of ultrasound image data 20 from the ultrasound probe 10 while the ultrasound probe 10 is moved along the skin surface 2a of the body portion 2. At least a subset of the ultrasound image data 20, 40 representing a plurality of ultrasound image frames 22 are input into a machine learning module 50, wherein the machine learning module 50 has been trained to determine a relative motion between ultrasound image frames 22. The machine learning module 50 determines an image motion path 60 indicating the relative motion between the ultrasound image frames 22. Finally, the motion path 64 determined by the pathing model 54 is merged with the image motion path 60 determined from the ultrasound image frames 22.

The merging of the motion path 64 and the image motion path 60 may be configured to determine the motion path 64 based on the image motion path 60 in many ways. The merging may be configured to perform verification, scaling, transformation, correction, or substitution of the optical flow data 24. For example, the image motion path 60 may be used as redundancy data in case the motion path 64 cannot be reliably determined under specific conditions. In the case where the distance measurement data 26 indicates that the optical flow sensor 24 is operating outside of the specified working height range, the merging may substitute a portion of optical flow data 24 with a portion of the image motion path 60 to determine a portion of the final trajectory.

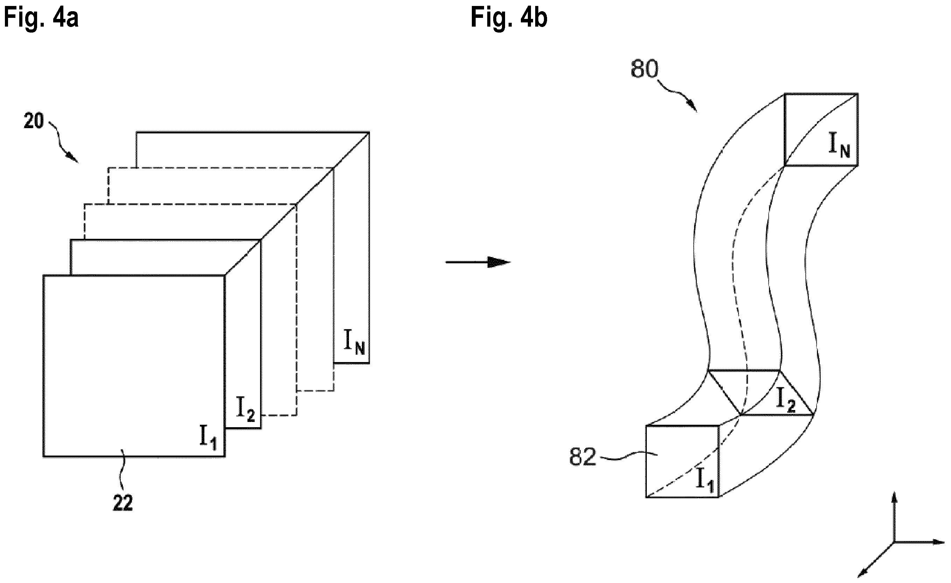

Figure 4a represents the input to the machine learning model 50, namely the ultrasound image data 20 (or a subset thereof) comprising a time series of ultrasound image frame data representing the ultrasound image frames 22. The ultrasound image data 20 may further include corresponding time information, such as a time stamp or time index. Alternatively, the acquisition of the ultrasound image data 20, the optical flow data 24, the distance measurement data 26 and optionally the inertial data 28 may be started concurrently with a standard acquisition interval so that data synchronisation can be achieved without specific time information. In addition, the ultrasound data 20 may also comprise metadata, e.g. indicating ultrasound settings and/or presets such as gain, frequency, and/or dynamic range of the ultrasound image frames 22. The metadata may also be partially or fully provided as a time series. In addition, the input to the machine learning model 50 may optionally include optical flow data 24, distance measurement data 26 and/or inertial data 28, and optionally a time series of the respective sensor data and corresponding time information, as described herein. Fig. 4b corresponds to Fig. 1b and the description of Fig. 1b above is also applicable to Fig. 4b.

Referring once again to Figure 5, the overall workflow of the second extension embodiment is shown, with the optional steps indicated with dashed lines now being included in the overall method. The main input of the sub-method for determining the image motion path 60 (shown on the right side of the flow-chart) is the ultrasound image data 20, or at least a subset thereof, generated by the ultrasound transducer 11 from the ultrasound probe 10. The term "at least a subset" requires that the information contained in the ultrasound image data from the ultrasound probe is at least partially input into the machine learning module 50. The ultrasound image data 20 may be pre-processed with a variety of algorithms in an image data pre-processing module 30, such as image resampling, image filtering or other high-level analysis. Generally, the (sub-set of) ultrasound image data 20 may be optionally pre-processed, filtered or altered in any other manner by the image data pre-processing module 30. The (pre-processed) ultrasound image data 20, 40 from multiple frames can then be input in a machine learning module 50 that is trained, from previously learned training data 52, to produce an image motion path 60 of the relative motion of the probe between the different input image frames.

The training from previously learned training data 52 is performed before its utilization and comprises adjusting the values of the model parameters so that its output values are as close as possible to the expected values, as is known in the art. In other words, the training comprises solving a minimization problem for minimizing a deviation functional (e.g., L2 norm) with respect to the expected values.

Such a process is repeated for all frames of the acquisition to produce an image motion path 60, output from the machine learning model 50. The motion path 64 as determined from the optical flow data 24, the distance measurement data 26 and optionally the inertial measurement data 28 is then merged with the image motion path 60. Optionally, the merged motion path data is then post-processed in a post-processing module 70 to produce the final trajectory of the probe 80.

Next, optional features of pre-processing of the ultrasound image data are described. According to an aspect, the method comprises pre-processing of the ultrasound image data before at least the subset of the ultrasound image data is input to the machine-learning module. For example, the pre-processing may include pre-computing a motion-indicative data. An example of motion-indicative data is the in-plane displacement data representing the in-plane displacement between the at least two of the ultrasound images. The method may then comprise inputting the motion-indicative data (such as the in-plane displacement data) as an additional input to the machine learning module 50. For example, motion-indicative data may be a two-dimensional data set such as a vector field, and may be input to the machine learning module 50 as an additional image channels.

An advantage of this feature is that by inputting to the machine-learning module 50 data representing explicitly some easily calculable aspects of the relative motion between frames, the machine-learning module 50 may be enabled to provide information on the remaining aspects more reliable and/or with fewer training data.

The pre-computing of the in-plane displacement may be carried out by any known method. According to an aspect, the pre-computing is carried out by an "optical flow" method according to the state of the art [see e.g. Gunnar Farneback, "Two-frame motion estimation based on polynomial expansion", Lecture Notes in Computer Science, 2003, (2749), 363-370]. Thus, the in-plane displacement data may be computed as an optical flow vector field representing a sub-pixel dense optical flow between the at least two ultrasound images.

Generally, the ultrasound image data 20 can be pre-processed by the image data pre-processing module 30 using at least one of the following:
- Resampling: The ultrasound image data 20 may be resampled to a given size or such that each of its pixels has a given resolution. This is done to make the system robust to some settings of the ultrasound system (like the depth or the number of scanlines used).
- Image Filtering: This includes any local filters (like low-pass or high-pass filters), adaptive filters (like speckle denoising, enhancing or masking) or global image transformation (like histogram equalization).
- Segmentation: Another pre-processing would consist in segmenting the image, i.e. classifying all pixels as one of multiple classes and using such probability maps as additional inputs. In a medical application for instance, an example would be to segment the skin, the fat, the muscle and the bone pixels.
- Any pre-computed feature: For instance, as described before, use as the optical flow vector field as additional channels for the model input.

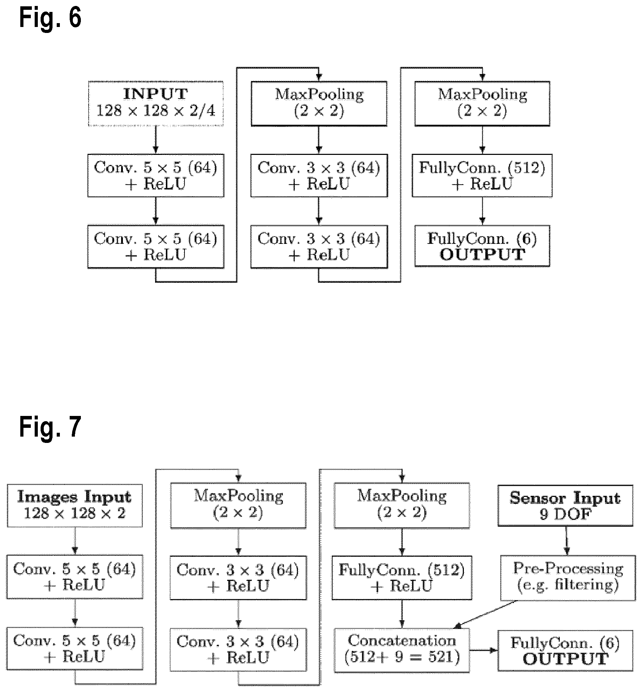

Next, exemplarily features describing the machine learning module 50 are discussed. The machine learning module 50 may comprise a neural network. Fig. 6 represents an example of a machine learning module 50 for use in embodiments of the invention, including a convolutional neural network (CNN). A two-channel image (representing two successive ultrasound frames) is the input of the neural network and goes through a series of convolutional layers (with 5x5 or 3x3 pixels kernels and 64 output channels), activation layers (here rectified linear units) and 2x2 pixels maximum pooling layers. At the end of the network, two fully connected layers aggregate the information from the whole features maps to a final output of six numbers representing 3 translations and 3 rotation parameters. These six numbers define an image motion path 60 determined from the ultrasound image data 20, which may be merged with the motion path 64 determined from the sensor data, and further used to parametrize the matrix M₁₂ mentioned above.

The parameters of the machine learning model 50 (here the convolution kernels and the coefficients of the fully connected layers) are set as the final state of the training process. Given a set of training data 52 (each training data sample can be composed of (i) a pair of successive ultrasound frames, and (ii) a very accurate estimate of the probe motion between those two frames, obtained for instance from a tracking system, and parameterized as six numbers), the training procedure can aim at minimizing the sum over all training data samples of the squared norm of the difference vector between the 6-dimensional output of the network and the 6 parameters of the actual measured probe motion. This minimization problem can be solved with a stochastic gradient descent or one of its variants like AdaGrad [John Duchi, Elad Hazan et Yoram Singer, "Adaptive subgradient methods for online learning and stochastic optimization", JMLR, vol. 12, 2011, p. 2121-2159] with a momentum of 90%, a batch size of 500 and no weight decay. The initial values of the network parameters can be randomly chosen, according to a Gaussian distribution with 0 mean and 0.01 standard deviation.

Optionally, an estimate of the in-plane translation can be pre-computed as the optical flow between the two images using known techniques (see e.g. Gunnar Farneback referenced above).

The output of this pre-computation of the optical flow is a 2D vector field that can be encoded as 2 additional optical flow channels. These 2 additional optical flow channels are used as additional input channels of the neural network forming the machine learning model 50 (in addition to the 2 image channels described above).

Optionally, the convolutional neural network includes a convolutional layer outputting a plurality of feature maps, each feature map being the result of a convolution with a particular kernel of the layer input. Throughout the present application, the indefinite article "a" is used in the sense of "at least one", and in particular includes the possibility of a plurality. The convolutional neural network may have a plurality of convolutional layers, e.g., two, three or four convolutional layers, connected to each other in series and optionally with a pooling layer between at least some of the convolutional layers.

The convolutional neural network may also include an activation layer (for instance a sigmoid or a rectified unit layer) and/or a fully connected layer that outputs either a global feature vector or the final prediction of the network. The convolutional neural network may, for example, comprise a plurality of (e.g. two) fully connected layers receiving input from the convolutional layer(s) and/or pooling layer(s), and providing as an output the motion data (e.g., six numbers representing 3 translations and 3 rotation parameters).

The neural network may be a recurrent neural network having a dynamic temporal behavior (i.e. the prediction of the network for a given ultrasound image data depends on the previous frames that have been inputted in the network). One popular architecture choice is for instance the long short-term memories (LSTM) networks.

Although the machine learning module 50 according to the invention has been mainly illustrated by a neural network, it is not limited to neural networks. Rather, other types of machine learning module 50 may also be used. For example, the machine learning module 50 may also include a random forest algorithm.

The machine learning module 50 may further obtain input data from other sources other than the ultrasound image data 20, such as external sensors. Similarly to Fig. 6, Fig. 7 represents an example of a neural network architecture that will take into account not only the ultrasound image data 20, but may also accept inertial data 28 obtained from the optional inertial measurement unit 18. The two architectures are mostly similar but the measurements of the inertial measurement unit 18 may be concatenated to the aggregated feature vector at the end of the network before producing the final output corresponding to the image motion path 60.

### Description of further aspects

Next, various more general aspects of the invention are defined in more detail. Each aspect so defined may be combined with any other embodiment or with any other aspect(s) unless clearly indicated to the contrary.

Referring once again to Fig. 3, according to an aspect of the present invention, an apparatus for determining a motion path of a movable ultrasound probe 10 is provided. The apparatus determines the motion path 64 of the movable ultrasound probe 10 during acquisition of an ultrasound image of a body portion 2 by the ultrasound probe 10. The apparatus includes an optical flow sensor 14 for obtaining a stream of optical flow data 24 indicative of a two-dimensional movement of the optical flow sensor 14 over a skin surface 2a of the body portion 2, a distance sensor 16 for obtaining distance measurement data 26 indicative of a distance D between the distance sensor 16 and the skin surface 2a of the body portion 2, a mounting structure 10a for fixedly mounting the optical flow sensor 14 and the distance sensor 16 to the ultrasound probe 10, and a motion determining module configured to determine, using a pathing model 54, the motion path based on the optical flow data 24 and the distance measurement data 26.

Particularly, the apparatus according to the above aspect is configured for carrying out the method for determining a motion path of an ultrasound probe according to aspects and embodiments described herein.

According to a further embodiment, which may be combined with other embodiments and aspects described herein, the apparatus may further include an inertial measurement unit 18 for obtaining a stream of inertial data 28 indicative of a rotation θx, θy, θz of the ultrasound probe, wherein the motion determining module is configured to determine the motion path 64 based further on the inertial data 28.

Particularly, the apparatus according to the above embodiment is configured for carrying out the method for determining a motion path of an ultrasound probe according to the first extension embodiment described herein, wherein the motion path 64 is further based on inertial data 28 from an inertial measurement unit 18.

According to a further embodiment, which may be combined with other embodiments and aspects described herein, the apparatus may further include a probe interface for receiving a stream of ultrasound image data 20 from the ultrasound probe 10 while the ultrasound probe 10 is moved along the skin surface 2a, and a machine learning module. The machine learning module 50 includes an input section adapted for receiving, as an input, at least a sub-set of the ultrasound image data representing a plurality of ultrasound image frames, and a training memory section containing a training memory having been trained to determine the relative motion between ultrasound image frames 22. The machine learning module is adapted for determining, from the input and using the training memory, an image motion path 60 indicating the relative motion between the ultrasound image frames 22. The apparatus further includes a merging system configured to merge the motion path 64 determined by the pathing model 54 and the image motion path 60 determined from the ultrasound image frames 22.

Particularly, the apparatus according to the above embodiment is configured for carrying out the method for determining a motion path of an ultrasound probe according to the second extension embodiment described herein, wherein the motion path 64 is further merged with an image motion path 60 determined from the ultrasound image data.

The exemplary apparatus shown in Fig. 3 is shown as a number of sensors arranged such that the sensors are fixedly arranged on the ultrasound probe 10. Particularly, an optical flow sensor 14, a distance sensor 16 and optionally an inertial measurement unit 18 are fixedly mounted on the ultrasound probe 10. As described above, the term "fixedly mounted" is not limited to the respective sensor being contained within a housing of the ultrasound probe 10 being a component of the ultrasound probe 10, or otherwise being permanently fixed to the ultrasound probe 10. Rather, the respective sensors may be configured to be mounted as a separate unit to the ultrasound probe 10 in either a permanent or a temporary manner, e.g. as a separate unit attached to the ultrasound probe 10 with clips or fasteners. Particularly, the optical flow sensor 14, distance sensor 16 and optionally the inertial measurement unit 18 may be housed within a mounting structure 10a which may be fixedly mounted to the ultrasound probe 10. This arrangement allows for the motion determining apparatus to be mounted to a variety of existing ultrasound probes, or for moving between different ultrasound probes.

According to aspects and embodiments of the present invention, an ultrasound probe 10 configured for ultrasound imaging of a body portion 2 is provided. The ultrasound probe 10 includes an ultrasound transducer 11, an optical flow sensor 14 configured for generating optical flow data 24 indicative of a two-dimensional movement over a skin surface 2a of the body portion 2, and a distance sensor configured for generating distance measurement data 26 indicative of a distance D between the distance sensor 16 and the skin surface 2a. The optical flow sensor may include an optical tracking sensor or a camera, and may have a working height between the skin surface 2a of the body portion 2 and the optical flow sensor 14 of at least 5 mm, particularly in the range of at least 5 mm to at most 50 mm, more particularly in the range of at least 15 mm to at most 35 mm. The distance sensor 16 may be a time-of-flight sensor, particularly an optical time-of-flight sensor, more particularly a laser distance sensor, and the distance sensor 16 may preferably be arranged for measuring the distance D with respect to a portion of the skin surface 2a within a field of view FOV of the optical flow sensor 14.

The ultrasound probe 10 according to the above aspect and embodiments is configured for acquiring ultrasound image data while having its motion path determined by the methods according to at least the main embodiment of the present invention. All relevant features and aspects discussed above for at least the main embodiment may be equally applied to the ultrasound probe according to these embodiments.

According to further embodiments, which may be combined with aspects and embodiments described herein, the ultrasound probe 10 may further include an inertial measurement unit 18 configured for sensing a rotation θx, θy, θz of the ultrasound probe 10. The ultrasound probe 10 according to this embodiment is configured for acquiring ultrasound image data while having its motion path determined by the methods according to at least the main embodiment and the first extension embodiment of the present invention. All relevant features and aspects discussed above for at least the main and first extension embodiment may be equally applied to the ultrasound probe according to this embodiment.

Once the above method for determining a motion path 64 of the ultrasound probe 10 is carried out, and a corresponding stream of ultrasound image data is acquired, the ultrasound image data may be processed based on the motion path 64 to obtain a three-dimensional tomographic image of the body portion. The motion path data, in combination with the ultrasound image frames acquired by the ultrasound probe 10, may be used in a reconstruction of the 3D volume of the body portion according to methods and processes known in the state of the art. The reconstruction may be carried out using artificial intelligence techniques, or machine learning techniques.

According to a further aspect, a method of generating a 3D tomographic image of a body portion 2 is provided. The method includes receiving a stream of ultrasound image data 20 from an ultrasound probe 10 while the ultrasound probe 10 is moved across a skin surface 2a of the body portion 2, receiving a stream of probe motion data of the ultrasound probe 10 by determining a motion path 64 according to the methods described in the present disclosure while the ultrasound probe 10 is moved across the skin surface 2a of the body portion 2, inputting at least a subset of the ultrasound image data 20, 40 representing a plurality of ultrasound image frames 22 into a processor, inputting at least a subset of the probe motion data representing the motion of the ultrasound probe 10 into the processor, and generating, by the processor, the 3D tomographic image based on the inputted ultrasound image data 20, 40 and the inputted probe motion data.

In a further embodiment, which may be combined with aspects and embodiments described herein, the processor includes a machine learning module which has been trained to generate 3D tomographic images based on the ultrasound image data 20, 40 and the probe motion data.

The processor used for generating the 3D tomographic image may include a computer, a controller, a microprocessor, a programmable logic controller (PLC), or a digital signal processor (DSP). Particularly, the processor may include a processing element, at least one input and at least one output, such that a data processing operation is performed on the at least one input and output to the at least one output. The processor may further include at least one storage means, which may include random access memory (RAM), read-only memory (ROM) and external data storage means such as hard disks, flash storage or network-attached storage.

While the present disclosure is directed to aspects and embodiments of the invention, other and further embodiments may be devised without departing from the basic scope thereof, and the scope thereof is determined by the claims that follow. In particular, this written description uses examples to disclose the disclosure, including the best mode, and also to enable any person skilled in the art to practice the described subject-matter, including making and using any devices or systems and performing any incorporated methods. While various specific embodiments have been disclosed in the present disclosure, mutually non-exclusive features of the embodiments described above may be combined with each other.

### Reference signs

- 2: Body portion
- 2a: Skin surface
- 10: Ultrasound probe
- 10a: Mounting structure
- 11: Ultrasound transducer
- 12: Scanning direction
- 14: Optical flow sensor
- 16: Distance sensor
- 18: Inertial measurement unit
- 20: Ultrasound image data
- 22: Ultrasound image frames
- 24: Optical flow data
- 26: Distance measurement data
- 28: Inertial data
- 30: Image data pre-processing module
- 34: Optical flow data pre-processing module
- 36: Distance measurement data pre-processing module
- 38: Inertial data pre-processing module
- 40: Pre-processed ultrasound image data
- 44: Pre-processed sensor data
- 50: Machine learning module
- 52: Training data
- 54: Pathing model
- 60: Image motion path
- 64: Motion path
- 70: Post-processing module
- 72: Verifying a consistency condition
- 74: Consistency condition
- 80: Post-processed trajectory data
- 82: Determined spatial arrangement of image frames
- I₁, I₂, ... I_{N}: Image frames
- C₁, C₂, ... C_{N}: Determined spatial arrangement of image frame coordinate systems
- M₁₂: Coordinate transformation function for image frame coordinate systems
- Δx, Δy: Differential data (in x-direction and y-direction, respectively)
- Σx, Σy: Summation data (in x-direction and y-direction, respectively)
- θx, θy, θz: Rotation of ultrasound probe (about x-axis, y-axis and z-axis, respectively)
- D: Distance between skin surface and distance sensor
- FOV: Field of view

## Claims

1. A method of determining a motion path (64) characterizing the motion of a movable ultrasound probe (10) across a skin surface (2a) of a body portion (2) during acquisition of an ultrasound image of the body portion (2) by the ultrasound probe (10), the method comprising:
receiving a stream of optical flow data (24) from an optical flow sensor (14) fixedly mounted to the ultrasound probe (10), wherein the optical flow data (24) is indicative of a two-dimensional movement of the optical flow sensor (14) over the skin surface (2a);
receiving distance measurement data (26) from a distance sensor (16) fixedly mounted to the ultrasound probe (10), wherein the distance measurement data (16) is indicative of a distance (D) between the distance sensor (16) and the skin surface (2a);
inputting the optical flow data (24) and the distance measurement data (26) into a pathing model (54); and
determining, by the pathing model (54), the motion path (64) based on the optical flow data (24) and the distance measurement data (26).

2. The method according to claim 1, wherein the pathing model (54) includes at least one of the group consisting of a linear scaling model, a non-linear scaling model, an empirical model based on empirical data, and a machine learning model.

3. The method according to any one of claims 1 to 2, wherein the motion path (64) includes at least one of the group comprising:
a scanning direction of the ultrasound probe (10) with respect to the skin surface (2a);
a scanning distance of the ultrasound probe (10) with respect to the skin surface (2a);
a yaw motion of the ultrasound probe (10) with respect to the skin surface (2a);
a scanning depth with respect to the body portion (2); and
a forward-backward directedness.

4. The method according to any one of claims 1 to 3, wherein the optical flow data (24) includes position differential data (Δx, Δy) and summation data (Σx, Σy) relative to the skin surface (2a).

5. The method according to any one of claims 1 to 4, wherein the distance sensor (16) is a time-of-flight sensor, particularly an optical time-of-flight sensor, more particularly a laser distance sensor, and wherein the distance sensor (16) is preferably arranged for measuring the distance (D) with respect to a portion of the skin surface (2a) within a field of view (FOV) of the optical flow sensor (14).

6. The method according to any one of claims 1 to 5, further comprising:
receiving inertial data (28) from an inertial measurement unit (18) fixedly mounted to the ultrasound probe (10), wherein the inertial data (28) is indicative of a rotation (θx, θy, θz) of the ultrasound probe (10),
wherein the determining, by the pathing model (54), of the motion path (64) is further based on the inertial data (28).

7. The method according to any one of claims 1 to 6 further comprising:
receiving a stream of ultrasound image data (20) from the ultrasound probe (10) while the ultrasound probe (10) is moved along the skin surface (2a) of the body portion (2);
inputting at least a subset of the ultrasound image data (20, 40) representing a plurality of ultrasound image frames (22) into a machine learning module (50), wherein the machine learning module (50) has been trained to determine a relative motion between ultrasound image frames (22);
determining, by the machine learning module (50), an image motion path (60) indicating the relative motion between the ultrasound image frames (22); and
merging the motion path (64) determined by the pathing model (54) and the image motion path (60) determined from the ultrasound image frames (22).

8. An apparatus for determining a motion path (64) of a movable ultrasound probe (10) during acquisition of an ultrasound image of a body portion (2) by the ultrasound probe (10), the apparatus comprising:
an optical flow sensor (14) for obtaining a stream of optical flow data (24) indicative of a two-dimensional movement of the optical flow sensor (14) over a skin surface (2a) of the body portion (2);
a distance sensor (16) for obtaining distance measurement data (26) indicative of a distance (D) between the distance sensor (16) and the skin surface (2a);
a mounting structure (10a) for fixedly mounting the optical flow sensor (14) and the distance sensor (16) to the ultrasound probe (10); and
a motion determining module configured to determine, using a pathing model (54), the motion path (64) based on the optical flow data (24) and the distance measurement data (26).

9. The apparatus of claim 8, further comprising:
an inertial measurement unit (18) for obtaining a stream of inertial data (28) indicative of a rotation (θx, θy, θz) of the ultrasound probe (10),
wherein the motion determining module is configured to determine the motion path (64) based further on the inertial data (28).

10. The apparatus of any one of claims 8 to 9, further comprising:
a probe input interface for receiving a stream of ultrasound image data (20) from the ultrasound probe (10) while the ultrasound probe (10) is moved along the skin surface (2a);
a machine-learning module (50) having
(a) an input section adapted for receiving, as an input, at least a sub-set of the ultrasound image data representing a plurality of ultrasound image frames (22),
(b) a training data section containing training data (52) having been trained to determine the relative motion between ultrasound image frames (22),
wherein the machine-learning module (50) is adapted for determining, from the input and using the training data (52), an image motion path (60) indicating the relative motion between the ultrasound image frames (22); and
a merging system configured to merge the motion path (64) determined by the pathing model (54) and the image motion path (60) determined from the ultrasound image frames (22).

11. An ultrasound probe (10) configured for ultrasound imaging of a body portion (2), comprising:
an ultrasound transducer (11);
an optical flow sensor (14) configured for generating optical flow data (24) indicative of a two-dimensional movement over a skin surface (2a) of the body portion (2); and
a distance sensor (16) configured for generating distance measurement data (26) indicative of a distance (D) between the distance sensor (16) and the skin surface (2a).

12. The ultrasound probe (10) according to claim 11, further comprising an inertial measurement unit (18) configured for sensing a rotation (θx, θy, θz) of the ultrasound probe (10).

13. The ultrasound probe (10) according to any one of claims 11 to 12, wherein the distance sensor (16) is a time-of-flight sensor, particularly an optical time-of-flight sensor, more particularly a laser distance sensor, and wherein the distance sensor (16) is preferably arranged for measuring the distance (D) with respect to a portion of the skin surface (2a) within a field of view (FOV) of the optical flow sensor (14).

14. A method of generating a 3D tomographic image of a body portion (2), comprising:
receiving a stream of ultrasound image data (20) from an ultrasound probe (10) while the ultrasound probe (10) is moved across a skin surface (2a) of the body portion (2);
receiving a stream of probe motion data of the ultrasound probe (10) by determining a motion path (64) according to the method of any one of claims 1 to 11 while the ultrasound probe (10) is moved across the skin surface (2a) of the body portion (2);
inputting at least a subset of the ultrasound image data (20, 40) representing a plurality of ultrasound image frames (22) into a processor;
inputting at least a subset of the probe motion data representing the motion of the ultrasound probe (10) into the processor; and
generating, by the processor, the 3D tomographic image based on the inputted ultrasound image data (20, 40) and the inputted probe motion data.

15. The method according to claim 14, wherein the processor includes a machine learning module which has been trained to generate 3D tomographic images based on the ultrasound image data (20, 40) and the probe motion data.

## Amended claims

### Amended claims in accordance with Rule 137(2) EPC.

1. A method of determining a motion path (64) characterizing the motion of a movable ultrasound probe (10) across a skin surface (2a) of a body portion (2) during acquisition of an ultrasound image of the body portion (2) by the ultrasound probe (10), the method comprising:
receiving a stream of optical flow data (24) from an optical flow sensor (14) fixedly mounted to the ultrasound probe (10), wherein the optical flow data (24) is indicative of a two-dimensional movement of the optical flow sensor (14) over the skin surface (2a), wherein the optical flow sensor (14) has a field of view (FOV) covering at least a portion of the skin surface (2a) which enables the measurement of the optical flow data (24) across the skin surface (2a);
receiving distance measurement data (26) from a distance sensor (16) fixedly mounted to the ultrasound probe (10), wherein the distance measurement data (16) is indicative of a distance (D) between the distance sensor (16) and the skin surface (2a);
inputting the optical flow data (24) and the distance measurement data (26) into a pathing model (54); and
determining, by the pathing model (54), the motion path (64) based on the optical flow data (24) and the distance measurement data (26).

2. The method according to claim 1, wherein the pathing model (54) includes at least one of the group consisting of a linear scaling model, a non-linear scaling model, an empirical model based on empirical data, and a machine learning model.

3. The method according to any one of claims 1 to 2, wherein the motion path (64) includes at least one of the group comprising:
a scanning direction of the ultrasound probe (10) with respect to the skin surface (2a);
a scanning distance of the ultrasound probe (10) with respect to the skin surface (2a);
a yaw motion of the ultrasound probe (10) with respect to the skin surface (2a);
a scanning depth with respect to the body portion (2); and
a forward-backward directedness.

4. The method according to any one of claims 1 to 3, wherein the optical flow data (24) includes position differential data (Δx, Δy) and summation data (Σx, Σy) relative to the skin surface (2a).

5. The method according to any one of claims 1 to 4, wherein the distance sensor (16) is a time-of-flight sensor, particularly an optical time-of-flight sensor, more particularly a laser distance sensor, and wherein the distance sensor (16) is preferably arranged for measuring the distance (D) with respect to a portion of the skin surface (2a) within the field of view (FOV) of the optical flow sensor (14).

6. The method according to any one of claims 1 to 5, further comprising:
receiving inertial data (28) from an inertial measurement unit (18) fixedly mounted to the ultrasound probe (10), wherein the inertial data (28) is indicative of a rotation (θx, θy, θz) of the ultrasound probe (10),
wherein the determining, by the pathing model (54), of the motion path (64) is further based on the inertial data (28).

7. The method according to any one of claims 1 to 6 further comprising:
receiving a stream of ultrasound image data (20) from the ultrasound probe (10) while the ultrasound probe (10) is moved along the skin surface (2a) of the body portion (2);
inputting at least a subset of the ultrasound image data (20, 40) representing a plurality of ultrasound image frames (22) into a machine learning module (50), wherein the machine learning module (50) has been trained to determine a relative motion between ultrasound image frames (22);
determining, by the machine learning module (50), an image motion path (60) indicating the relative motion between the ultrasound image frames (22); and
merging the motion path (64) determined by the pathing model (54) and the image motion path (60) determined from the ultrasound image frames (22).

8. An ultrasound probe (10) configured for ultrasound imaging of a body portion (2), comprising:
an ultrasound transducer (11);
an optical flow sensor (14) configured for generating optical flow data (24) indicative of a two-dimensional movement over a skin surface (2a) of the body portion (2), wherein the optical flow sensor (14) has a field of view (FOV) covering at least a portion of the skin surface (2a) which enables the measurement of the optical flow data (24) across the skin surface (2a); and
a distance sensor (16) configured for generating distance measurement data (26) indicative of a distance (D) between the distance sensor (16) and the skin surface (2a).

9. The ultrasound probe (10) according to claim 8, further comprising an inertial measurement unit (18) configured for sensing a rotation (θx, θy, θz) of the ultrasound probe (10).

10. The ultrasound probe (10) according to any one of claims 8 to 9, wherein the distance sensor (16) is a time-of-flight sensor, particularly an optical time-of-flight sensor, more particularly a laser distance sensor, and wherein the distance sensor (16) is preferably arranged for measuring the distance (D) with respect to a portion of the skin surface (2a) within the field of view (FOV) of the optical flow sensor (14).

11. An apparatus for determining a motion path (64) of a movable ultrasound probe (10) according to any one of claims 8 to 10, during acquisition of an ultrasound image of a body portion (2) by the ultrasound probe (10), the apparatus comprising:
the optical flow sensor (14);
the distance sensor (16);
a mounting structure (10a) for fixedly mounting the optical flow sensor (14) and the distance sensor (16) to the ultrasound probe (10); and
a motion determining module configured to determine, using a pathing model (54), the motion path (64) based on the optical flow data (24) and the distance measurement data (26).

12. The apparatus of claim 11, further comprising:
an inertial measurement unit (18) for obtaining a stream of inertial data (28) indicative of a rotation (θx, θy, θz) of the ultrasound probe (10),
wherein the motion determining module is configured to determine the motion path (64) based further on the inertial data (28).

13. The apparatus of any one of claims 11 to 12, further comprising:
a probe input interface for receiving a stream of ultrasound image data (20) from the ultrasound probe (10) while the ultrasound probe (10) is moved along the skin surface (2a);
a machine-learning module (50) having
(a) an input section adapted for receiving, as an input, at least a sub-set of the ultrasound image data representing a plurality of ultrasound image frames (22),
(b) a training data section containing training data (52) having been trained to determine the relative motion between ultrasound image frames (22),
wherein the machine-learning module (50) is adapted for determining, from the input and using the training data (52), an image motion path (60) indicating the relative motion between the ultrasound image frames (22); and
a merging system configured to merge the motion path (64) determined by the pathing model (54) and the image motion path (60) determined from the ultrasound image frames (22).

14. A method of generating a 3D tomographic image of a body portion (2), comprising:
receiving a stream of ultrasound image data (20) from an ultrasound probe (10) while the ultrasound probe (10) is moved across a skin surface (2a) of the body portion (2);
receiving a stream of probe motion data of the ultrasound probe (10) by determining a motion path (64) according to the method of any one of claims 1 to 7 while the ultrasound probe (10) is moved across the skin surface (2a) of the body portion (2);
inputting at least a subset of the ultrasound image data (20, 40) representing a plurality of ultrasound image frames (22) into a processor;
inputting at least a subset of the probe motion data representing the motion of the ultrasound probe (10) into the processor; and
generating, by the processor, the 3D tomographic image based on the inputted ultrasound image data (20, 40) and the inputted probe motion data.

15. The method according to claim 14, wherein the processor includes a machine learning module which has been trained to generate 3D tomographic images based on the ultrasound image data (20, 40) and the probe motion data.
